# EUROPEAN PATENT SPECIFICATION

(11) **EP 2 769 725 B1**
(45) Date of publication and mention of the grant of the patent: **29.06.2016**
(21) Application number: 14155548.2
(22) Date of filing: 18.02.2014
(51) Int. Cl.: A61K 31/683, A61K 33/08, A61K 31/194, A61K 31/20, A61P 15/00, A23L 33/00

(54) **Compositions for use in alleviating symptoms associated with premenstrual syndrome and premenstrual dysphoric disorder**
Zusammensetzungen zur Linderung von Symptomen im Zusammenhang mit dem prämenstruellen Syndrom und prämenstrueller Dysphoriestörung
Compositions destinées à être utilisées pour soulager les symptômes associés aux syndrome prémenstruel et trouble dysphorique prémenstruel

(30) Priority: 22.02.2013 US 201313773653
(43) Date of publication of application: 27.08.2014
(73) Proprietor: Lipogen Ltd., 34372 Haifa (IL)
(72) Inventor: Rutenberg, David, 34372 Haifa (IL); Perry Faierwerger, Rina, 30992 Moshav Bat Shlomo (IL)
(74) Representative: Modiano, Micaela Nadia

(56) References cited:
- EP-A1- 1 661 575
- EP-A1- 2 322 184
- EP-A2- 1 201 244
- US-B2- 8 324 187
- MONTANÉ AND PÉREZ-BALILESTER: "Cyclic changes in phospholipid content and composition in human endometrium during the menstrual cycle", JOURNAL OF REPRODUCTION AND FERTILITY, vol. 73, no. 2, March 1985 (1985-03), pages 317-321, XP008177969,

## Description

### FIELD AND BACKGROUND OF THE INVENTION

The present invention relates to compositions for use in alleviating symptoms associated with premenstrual syndrome (PMS) and premenstrual dysphoric disorder (PMDD).

The U.S. Department of Health and Human Services, Office on Women's Health, summarizes that PMS is a group of symptoms linked to the menstrual cycle. PMS symptoms occur in the week or two weeks prior to menstruation. PMS may be just a monthly bother, or it may be so severe that it makes it hard to even get through the day. Up to 80% of women experience some symptoms of PMS. The causes of PMS are not clear, and are linked to hormonal changes during the menstrual cycle. Stress and emotional problems do not seem to cause PMS.

The inclusion of PMDD in the fifth edition of the Diagnostic and Statistical Manual of Mental Disorders (DSM-5, due for release in 2013) is indicative of the dynamic and controversial attitude toward PMDD as a full-fledged disorder within the scientific community. PMDD was previously considered to be synonymous with PMS. The scientific community later differentiated PMDD as a subset of symptoms associated with PMS, and more recently as a specific type of occurrence of PMS. However, PMDD is not viewed as a unique disorder globally, particularly in Europe.

In a scientific article by Epperson et al. (Am J Psychiatry, 2012 May;169(5):465-75) entitled, "Premenstrual Dysphoric Disorder: Evidence for a New Category for DSM-5," the abstract states, "Based on thorough review and lengthy discussion, the work group proposed that the information on the diagnosis, treatment, and validation of the disorder has matured sufficiently for it to qualify as a full category in DSM-5."

This clearly suggests a shift has occurred in the state-of-the-art understanding of PMDD vis-à-vis PMS. That is, while PMDD was once considered to be synonymous with PMS, it later came to be commonly understood as an offshoot of PMS, exhibiting a similar rubric of symptoms associated with menstruation. The inclusion of PMDD in the DSM-5 further validates the recognition of such a "subset" disorder as PMDD as a stand-alone mental disorder.

The prior art, European Patent Application No. EP 2322184 discloses pharmaceutical/nutritional compositions for alleviating symptoms associated with premenstrual syndrome (PMS) including: at least 2% (w/w) phosphatidyl-L-serine, or salts thereof, out of a total composition, as an effective ingredient, wherein: the phosphatidyl-L-serine has a structural fatty-acid chain derived from at least one raw material lecithin.

It would be desirable to have compositions for use in alleviating symptoms associated with PMS and PMDD.

### SUMMARY OF THE INVENTION

It is the purpose of the present invention to provide compositions for use in alleviating symptoms associated with PMS and PMDD.

Furthermore, it is noted that the term "exemplary" is used herein to refer to examples of embodiments and/or implementations, and is not meant to necessarily convey a more-desirable use-case. Similarly, the term "preferred" is used herein to refer to an example out of an assortment of contemplated embodiments and/or implementations, and is not meant to necessarily convey a more-desirable use-case. Therefore, it is understood from the above that "exemplary" and "preferred" may be applied herein to multiple embodiments and/or implementations.

Embodiments of the present invention provide compositions for use in decreasing PMS and PMDD symptoms, including phosphatidic acid and a bio-available form of magnesium. Such compositions are administrable via intravenous or oral administration. Such compositions can also include other excipients (e.g., additional phospholipids, lyso-phospholipids, sugars, and proteins) to prepare capsules, tablets, and granules with improved handling and shelf life. Because of the absence of any safety problem, such compositions can be blended into daily foods and beverages, either in powder or liquid form, or as a hydrogenated substance for use in decreasing PMS/PMDD symptoms. Embodiments of the present invention further provide compositions including phosphatidic acid.

Therefore, according to the present invention, there is provided for the first time a pharmaceutical/nutritional composition for alleviating symptoms associated with premenstrual syndrome (PMS) and premenstrual dysphoric disorder (PMDD), the pharmaceutical/nutritional composition including: (a) at least 2% (w/w) phosphatidic acid, or salts thereof, out of the total effective composition, as a first effective ingredient; and (b) a suitable amount of at least one bio-available form of magnesium as a second effective ingredient.

Preferably, at least one bio-available form is selected from the group consisting of: magnesium oxide, magnesium citrate, magnesium hydroxide, and magnesium stearate.

Preferably, at least one bio-available form is a magnesium salt of the phosphatidic acid.

Preferably, the pharmaceutical/nutritional composition further includes: (c) a pharmaceutical excipient.

Preferably, the pharmaceutical/nutritional composition further includes: (c) a nutritional excipient.

Preferably, the total effective composition is administrable in a multi-part regimen.

Preferably, the total effective composition is administrable by at least one delivery method selected from the group consisting of: oral delivery and intravenous delivery.

These and further embodiments will be apparent from the detailed description and examples that follow.

### DESCRIPTION OF THE PREFERRED EMBODIMENTS

The present invention relates to compositions for use in alleviating symptoms associated with PMS and PMDD. The aspects, uses, and advantages for such compositions, according to the present invention, may be better understood with reference to the accompanying description. Exemplary embodiments of the present invention are detailed below in the following exemplary formulations.

Pharmaceutical/nutritional compositions for use in alleviating symptoms associated with PMS and PMDD were formulated using effective amounts of phosphatidyl-L-serine (reference examples) and at least one bio-available form of magnesium. Some formulations also included phosphatidic acid.

### EXEMPLARY FORMULATON A: (reference example)

Phosphatidyl-L-serine (PS) was prepared by Lipogen Products (9000) Ltd. via the process of enzymatic reaction from a substrate soybean lecithin using the method described in Example 1-1 of the parent patent application by Rutenberg et al. (US Patent Publication No. 2011/0098249, hereinafter referred to as Rutenberg '249) of the present invention. Magnesium oxide (Dr. Paul Lohmann GmbH KG) was used as a bio-available form of magnesium. 250g. of PS were combined with 250g. of magnesium oxide to produce the formulation.

### EXEMPLARY FORMULATON B: (reference example)

PS was prepared by Lipogen Products (9000) Ltd. as in Formulation A. PS was then converted into a magnesium salt by ion exchange with magnesium chloride (Dr. Paul Lohmann GmbH KG). The magnesium salt of PS (PS-Mg) was used as a bio-available form of magnesium. 200g. of PS-Mg were used to produce the formulation.

### EXEMPLARY FORMULATON C:

PS was prepared by Lipogen Products (9000) Ltd. as in Formulation A. Magnesium citrate (Dr. Paul Lohmann GmbH KG) was used as a bio-available form of magnesium. Phosphatidic acid (PA) was prepared by Lipogen Products (9000) Ltd.). 250g. of PS were combined with 250g. of PA and 250g. of magnesium citrate to produce the formulation.

### EXEMPLARY FORMULATON D:

PS and PA were prepared by Lipogen Products (9000) Ltd. as in Formulation C. PA was then converted into a magnesium salt by ion exchange with magnesium chloride (Dr. Paul Lohmann GmbH KG). The magnesium salt of PA (PA-Mg) was used as a bio-available form of magnesium. 200g. of PS were combined with 200g. of PA-Mg to produce the formulation.

### EXEMPLARY FORMULATON E:

PA-Mg was prepared by Lipogen Products (9000) Ltd. as in Formulation D. 200g. of PA-Mg were used to produce the formulation.

### EXEMPLARY FORMULATON F:

PA was prepared by Lipogen Products (9000) Ltd. as in Formulation C. 500g. of PA were used to produce the formulation. Magnesium citrate (Dr. Paul Lohmann GmbH KG) was used as a bio-available form of magnesium. 200g. of PA were combined with 200g. of magnesium citrate to produce the formulation.

### EXEMPLARY FORMULATON G: (reference example)

PS was prepared by Lipogen Products (9000) Ltd. as in Formulation A. 500g. of PS were used to produce the formulation.

### EXEMPLARY FORMULATON H:

PA was prepared by Lipogen Products (9000) Ltd. as in Formulation C. 500g.
of PA were used to produce the formulation.

### RESULTS:

The effect of alleviating PMS/PMDD symptoms via oral administration was investigated in the following experiments. In studies involving PS/Mg formulations, the results are presented relative to the studies involving only PS (Formulation G - reference example) which were disclosed in Rutenberg '249. In studies involving PA/Mg formulations, the results are presented relative to studies described below involving only PA (Formulation H). It is noted that studies using Formulation H involving PA (Treatment H) showed results comparable to Formulation G (reference example) involving PS relative to a baseline of no treatment.

The PMS/PMDD symptom scale used was based on an assessment by the subject. Examples of the PMS/PMDD physical symptoms include acne, breast swelling and tenderness, feeling tired, having trouble sleeping, upset stomach, bloating, weight gain, constipation, diarrhea, headache, heart palpitations, backache, appetite changes, food cravings, and joint and muscle pain.

The PMS/PMDD behavioral symptoms include any changes that the subjects noticed in everyday behavior. Examples of the PMS/PMDD behavioral symptoms include feelings of deep sadness or despair, feelings of intense tension or anxiety, increased intense sensitivity to rejection or criticism, panic attacks, rapid and severe mood swings, uncontrollable crying, lasting irritability or anger, apathy, difficulty concentrating, chronic fatigue, severe insomnia or hypersomnia, feeling overwhelmed, feeling out of control, change in sex drive, and increased need for emotional closeness.

Classification of the subjects as suffering from PMDD was based on the presence of five or more of the above symptoms, with the presence of mood symptoms typically being dominant.

The results correlate to the following subjective ranking: "✔" = comparable improvement to PS- or PA-only treatments (Formulations G or H, respectively), "*" = slight improvement above treatments with Formulations G or H, and "**" = large improvement above treatments with Formulations G or H.

Formulation A: Three female volunteers who normally suffer from PMS symptoms and four females who suffer from PMDD received 200mg. of Formulation A (reference example) four times per day from three weeks before the expected monthly menstruation until the commencement of menstruation (Treatment A). The results are presented in Table 1. As indicated in Table 1, a significant improvement was observed in all seven participants, irrespective of age and malady of PMS versus PMDD.

**Table 1. The effect of alleviating PMS/PMDD symptoms using a PS/Mg treatment in an initial treatment regimen experiment (reference example).**

| Cumulative symptoms with Treatment A | Behavioral symptoms with Treatment A | Physical symptoms with Treatment A | Subject age suffering from) (PMS or PMDD |
|---|---|---|---|
| ** | * | * | (PMS) 35 |
| * | * | * | (PMS) 32 |
| * | ✔ | * | (PMS) 34 |
| ** | ** | * | (PMDD) 27 |
| ** | ** | * | (PMDD) 29 |
| * | * | * | (PMDD) 28 |
| * | * | * | (PMDD) 32 |

After one menstruation cycles after the menstruation date of the study in Table 1, the same seven females who participated reported that after cessation of the treatment, their usual symptoms reappeared. The above seven female then received 200mg. of Formulation A (reference example) four times per day from three weeks before the expected monthly menstruation until the commencement of menstruation (Treatment A). The results are presented in Table 2. As indicated in Table 1, a significant improvement was observed in all seven participants, irrespective of age and malady of PMS versus PMDD.

**Table 2. The effect of alleviating PMS/PMDD symptoms using a PS/Mg treatment in a secondary treatment regimen experiment after the re-emergence of symptoms (reference example).**

| Cumulative symptoms with Treatment A | Behavioral symptoms with Treatment A | Physical symptoms with Treatment A | Subject age suffering from) (PMS or PMDD |
|---|---|---|---|
| ** | * | * | (PMS) 35 |
| * | * | * | (PMS) 32 |
| ** | ✔ | ** | (PMS) 34 |
| ** | ** | * | (PMDD) 27 |
| ** | ** | * | (PMDD) 29 |
| * | ✔ | * | (PMDD) 28 |
| * | ✔ | * | (PMDD) 32 |

Formulation B: Three female volunteers who normally suffer from PMS symptoms and four females who suffer from PMDD received 150mg. of Formulation B (reference example) four times per day from three weeks before the expected monthly menstruation until the commencement of menstruation (Treatment B). The results are presented in Table 3. As indicated in Table 3, a significant improvement was observed in all seven participants, irrespective of age and malady of PMS versus PMDD.

**Table 3. The effect of alleviating PMS/PMDD symptoms using a PS/Mg treatment in an initial treatment regimen experiment (reference example).**

| Cumulative symptoms with Treatment B | Behavioral symptoms with Treatment B | Physical symptoms with Treatment B | Subject age suffering from) (PMS or PMDD |
|---|---|---|---|
| ** | * | * | (PMS) 35 |
| ** | * | ** | (PMS) 32 |
| * | ✔ | * | (PMS) 34 |
| ** | ** | * | (PMDD) 27 |
| ** | ** | ** | (PMDD) 29 |
| * | * | * | (PMDD) 28 |
| ** | ** | * | (PMDD) 32 |

Formulation C: Three female volunteers who normally suffer from PMS symptoms and four females who suffer from PMDD received 300mg. of Formulation C four times per day from three weeks before the expected monthly menstruation until the commencement of menstruation (Treatment C). The results are presented in Table 4. As indicated in Table 4, a significant improvement was observed in all seven participants, irrespective of age and malady of PMS versus PMDD.

**Table 4. The effect of alleviating PMS/PMDD symptoms using a PS/PA/Mg treatment in an initial treatment regimen experiment.**

| Cumulative symptoms with Treatment C | Behavioral symptoms with Treatment C | Physical symptoms with Treatment C | Subject age suffering from) (PMS or PMDD |
|---|---|---|---|
| ** | * | ** | (PMS) 35 |
| ** | ** | ** | (PMS) 32 |
| ** | ✔ | ** | (PMS) 34 |
| ** | ** | * | (PMDD) 27 |
| ** | ** | ** | (PMDD) 29 |
| * | ** | * | (PMDD) 28 |
| ** | * | ** | (PMDD) 32 |

Formulation D: Three female volunteers who normally suffer from PMS symptoms and four females who suffer from PMDD received 250mg. of Formulation D four times per day from three weeks before the expected monthly menstruation until the commencement of menstruation (Treatment D). The results are presented in Table 5. As indicated in Table 5, a significant improvement was observed in all seven participants, irrespective of age and malady of PMS versus PMDD.

**Table 5. The effect of alleviating PMS/PMDD symptoms using a PS/PA/Mg treatment in an initial treatment regimen experiment.**

| Cumulative symptoms with Treatment D | Behavioral symptoms with Treatment D | Physical symptoms with Treatment D | Subject age suffering from) (PMS or PMDD |
|---|---|---|---|
| ** | * | ** | (PMS) 35 |
| * | * | ** | (PMS) 32 |
| * | * | * | (PMS) 34 |
| ** | ** | ** | (PMDD) 27 |
| ** | ** | * | (PMDD) 29 |
| * | * | * | (PMDD) 28 |
| ** | * | ** | (PMDD) 32 |

Formulation E: Three female volunteers who normally suffer from PMS symptoms and four females who suffer from PMDD received 125mg. of Formulation E four times per day from three weeks before the expected monthly menstruation until the commencement of menstruation (Treatment E). The results are presented in Table 6. As indicated in Table 6, a significant improvement was observed in all seven participants, irrespective of age and malady of PMS versus PMDD.

**Table 6. The effect of alleviating PMS/PMDD symptoms using a PA/Mg treatment in an initial treatment regimen experiment.**

| Cumulative symptoms with Treatment E | Behavioral symptoms with Treatment E | Physical symptoms with Treatment E | Subject age suffering from) (PMS or PMDD |
|---|---|---|---|
| ** | * | * | (PMS) 35 |
| * | * | * | (PMS) 32 |
| * | ✔ | * | (PMS) 34 |
| * | * | * | (PMDD) 27 |
| ** | ** | * | (PMDD) 29 |
| ** | * | ** | (PMDD) 28 |
| * | * | * | (PMDD) 32 |

Formulation F: Three female volunteers who normally suffer from PMS symptoms and four females who suffer from PMDD received 200mg. of Formulation F four times per day from three weeks before the expected monthly menstruation until the commencement of menstruation (Treatment F). The results are presented in Table 7. As indicated in Table 7, a significant improvement was observed in all seven participants, irrespective of age and malady of PMS versus PMDD.

**Table 7. The effect of alleviating PMS/PMDD symptoms using a PA/Mg treatment in an initial treatment regimen experiment.**

| Cumulative symptoms with Treatment F | Behavioral symptoms with Treatment F | Physical symptoms with Treatment F | Subject age suffering from) (PMS or PMDD |
|---|---|---|---|
| * | * | * | (PMS) 35 |
| ** | * | ** | (PMS) 32 |
| * | ✔ | * | (PMS) 34 |
| ** | ** | * | (PMDD) 27 |
| ** | ** | * | (PMDD) 29 |
| ** | * | ** | (PMDD) 28 |
| * | * | * | (PMDD) 32 |

The treatments can be continuously and readily administered with no pain because the PS, PA, and Mg supplied in the compositions described above are freely ingested.

## Claims

1. A pharmaceutical/nutritional composition for use in alleviating symptoms associated with premenstrual syndrome (PMS) and premenstrual dysphoric disorder (PMDD), the pharmaceutical/nutritional composition comprising:
(a) at least 2% (w/w) phosphatidic acid, or salts thereof, out of said total effective composition, as a first effective ingredient; and
(b) a suitable amount of at least one bio-available form of magnesium as a second effective ingredient.

2. The pharmaceutical/nutritional composition for use according to claim 1, wherein said at least one bio-available form is selected from the group consisting of: magnesium oxide, magnesium citrate, magnesium hydroxide, and magnesium stearate.

3. The pharmaceutical/nutritional composition for use according to claim 1, wherein said at least one bio-available form is a magnesium salt of said phosphatidic acid.

4. The pharmaceutical/nutritional composition for use according to claim 1, the pharmaceutical/nutritional composition further comprising:
(c) a pharmaceutical excipient.

5. The pharmaceutical/nutritional composition for use according to claim 1, the pharmaceutical/nutritional composition further comprising:
(c) a nutritional excipient.

6. The pharmaceutical/nutritional composition for use according to claim 1, wherein said total effective composition is administrable in a multi-part regimen.

7. The pharmaceutical/nutritional composition for use according to claim 1, wherein said total effective composition is administrable by at least one delivery method selected from the group consisting of: oral delivery and intravenous delivery.

## Patentansprüche

1. Eine pharmazeutische/Ernährungs- Zusammensetzung für die Verwendung in der Linderung von Symptomen, die mit prämenstruellem Syndrom (PMS) und prämenstrueller dysphorischer Störung (PMDD) assoziiert sind, wobei die pharmazeutische/Ernährungs- Zusammensetzung folgendes umfasst:
(a) mindestens 2% (m/m) von der gesamten wirksamen Zusammensetzung Phosphatidsäure, oder Salze davon, als einen ersten wirksamen Inhaltsstoff; und
(b) eine geeignete Menge mindestens einer bioverfügbaren Form von Magnesium als einen zweiten wirksamen Inhaltsstoff.

2. Die pharmazeutische/Ernährungs- Zusammensetzung für die Verwendung gemäß Anspruch 1, worin die mindestens eine bioverfügbare Form gewählt ist aus der Gruppe bestehend aus: Magnesiumoxid, Magnesiumcitrat, Magnesiumhydroxid, und Magnesiumstearat.

3. Die pharmazeutische/Ernährungs- Zusammensetzung für die Verwendung gemäß Anspruch 1, worin die mindestens eine bioverfügbare Form ein Magnesiumsalz der Phosphatidsäure ist.

4. Die pharmazeutische/Ernährungs- Zusammensetzung für die Verwendung gemäß Anspruch 1, wobei die pharmazeutische/Ernährungs- Zusammensetzung weiter folgendes umfasst:
(c) einen pharmazeutischen Hilfsstoff.

5. Die pharmazeutische/Ernährungs- Zusammensetzung für die Verwendung gemäß Anspruch 1, wobei die pharmazeutische/Ernährungs- Zusammensetzung weiter folgendes umfasst:
(c) einen Ernährungs-Hilfsstoff.

6. Die pharmazeutische/Ernährungs- Zusammensetzung für die Verwendung gemäß Anspruch 1, worin die gesamte wirksame Zusammensetzung in einem mehrteiligen Regime verabreichbar ist.

7. Die pharmazeutische/Ernährungs- Zusammensetzung für die Verwendung gemäß Anspruch 1, worin die gesamte wirksame Zusammensetzung durch mindestens ein Zuführungsverfahren verabreichbar ist, gewählt aus der Gruppe bestehend aus; oraler Zuführung und intravenöser Zuführung.

## Revendications

1. Composition pharmaceutique/nutritionnelle pour utilisation pour soulager les symptômes associés au syndrome prémenstruel (PMS) et au trouble dysphorique prémenstruel (PMDD), la composition pharmaceutique/nutritionnelle comprenant:
(a) au moins 2% (p/p) d'acide phosphatidique, ou les sels de celui-ci, sur ledit total, de la composition efficace, en tant que premier ingrédient actif; et
(b) une quantité appropriée d'au moins une forme de magnésium bio-disponible en tant que deuxième ingrédient actif.

2. Composition pharmaceutique/nutritionnelle pour utilisation selon la revendication 1, dans laquelle ladite au moins une forme bio-disponible est choisie dans le groupe constitué par: l'oxyde de magnésium, le citrate de magnésium, l'hydroxyde de magnésium et le stéarate de magnésium.

3. Composition pharmaceutique/nutritionnelle pour utilisation selon la revendication 1, dans laquelle ladite au moins une forme bio-disponible est un sel de magnésium dudit acide phosphatidique.

4. Composition pharmaceutique/nutritionnelle pour utilisation selon la revendication 1, la composition pharmaceutique/nutritionnelle comprenant en outre:
(c) un excipient pharmaceutique.

5. Composition pharmaceutique/nutritionnelle pour utilisation selon la revendication 1, la composition pharmaceutique/nutritionnelle comprenant en outre:
(c) un excipient nutritionnel,

6. Composition pharmaceutique/nutritionnelle pour utilisation selon la revendication 1, dans laquelle ladite composition efficace totale peut être administrée selon un régime en plusieurs parties.

7. Composition pharmaceutique/nutritionnelle pour utilisation selon la revendication 1, dans laquelle ladite composition efficace totale peut être administrée selon au moins un mode d'administration choisi dans le groupe constitué par: l'administration par voie orale et l'administration par voie intraveineuse.
